(19) Europäisches Patentamt
European Patent Office
Office européen des brevets

(11) EP 4 667 023 A1

(12) EUROPEAN PATENT APPLICATION
published in accordance with Art. 153(4) EPC

(43) Date of publication:
**24.12.2025 Bulletin 2025/52**

(21) Application number: **24807045.0**

(22) Date of filing: **30.04.2024**

(51) International Patent Classification (IPC):
***A61L 29/02*** *(2006.01)* ***A61L 29/06*** *(2006.01)*
***A61L 29/14*** *(2006.01)* ***C08K 3/11*** *(2018.01)*
***C08K 5/29*** *(2006.01)* ***C08L 77/00*** *(2006.01)*

(52) Cooperative Patent Classification (CPC):
**A61L 29/02; A61L 29/06; A61L 29/14; C08K 3/11; C08K 5/29; C08L 77/00**

(86) International application number:
**PCT/JP2024/016709**

(87) International publication number:
**WO 2024/237091 (21.11.2024 Gazette 2024/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **17.05.2023 JP 2023081659**

(71) Applicant: **Terumo Kabushiki Kaisha**
**Tokyo 151-0072 (JP)**

(72) Inventor: **WATANABE, Nozomu**
**Fujinomiya-shi, Shizuoka 418-0015 (JP)**

(74) Representative: **KIPA AB**
**Drottninggatan 11**
**252 21 Helsingborg (SE)**

# (54) CATHETER TIP

(57) There is provided a catheter that is unlikely to deteriorate even after long-term storage. A catheter tip comprising: a resin having an ester bond and/or an amide bond; a tungsten-containing compound; and a hydrolysis inhibitor.

FIG. 1

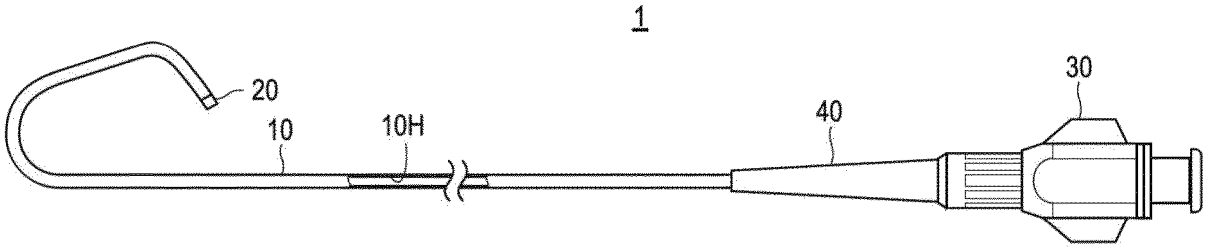


EP 4 667 023 A1

## Description

### Technical Field

The present invention relates to a catheter tip.

### Background Art

When performing treatment or diagnosis of a stenosed site, a catheter is used to insert a medical device into the living body and guide it to the target location. When inserting a catheter into the living body, in order to accurately identify the position of the distal end of the catheter, the distal end portion of the catheter (catheter tip) typically contains an X-ray opaque material that enables detection by X-rays. For example, metallic tungsten powder is known as an excellent X-ray opaque material and is used as a material for the distal end portion of the catheter (for example, refer to Patent Literature 1).

### Citation List

### Patent Literature

Patent Literature 1: US Patent No. 5584821

### Summary of Invention

Catheters are not always used immediately and may sometimes be stored for a long period before use. Resin is typically used in catheter tips for moldability and flexibility. However, when tungsten is incorporated into the catheter tip as an X-ray opaque material, there is a risk that the resin may deteriorate.
Accordingly, an object of the present invention is to provide a catheter tip in which the resin is less likely to deteriorate even after long-term storage, and a catheter including the same.
A catheter tip according to one aspect of the present invention has the following configuration.

(1) A catheter tip including: a resin having an ester bond and/or an amide bond; a tungsten-containing compound; and a hydrolysis inhibitor.
(2) The catheter tip according to (1), in which the hydrolysis inhibitor is a carbodiimide group-containing compound.
(3) The catheter tip according to (1) or (2), in which the content of the tungsten-containing compound is 10 to 90% by mass relative to a total amount of the resin and the tungsten-containing compound.
(4) The catheter tip according to any one of (1) to (3), in which the resin having an ester bond and/or an amide bond includes a polyamide-based resin.
(5) The catheter tip according to any one of (1) to (4), in which the tungsten-containing compound is dispersed as particles within the resin.
(6) A catheter comprising the catheter tip according to any one of (1) to (5).

### Brief Description of Drawings

Fig. 1 is a diagram illustrating an overall configuration of a catheter.
Fig. 2 is a cross-sectional schematic diagram of the vicinity of a junction of a catheter tip and a catheter main body portion.

### Description of Embodiments

The present invention provides a catheter tip including a resin having an ester bond and/or an amide bond; a tungsten-containing compound; and a hydrolysis inhibitor.
According to the catheter tip and the catheter including the same of the present invention, the risk of resin deterioration is reduced even after long-term storage.
The tungsten-containing compound is usually used in combination with a resin. The present inventor speculated that the cause of resin deterioration due to long-term storage may be the hydrolysis of ester bond and/or amide bond sites in the presence of the tungsten-containing compound. As a result of studies based on this, it was found that the aging deterioration of the resin can be suppressed by using a hydrolysis inhibitor in combination, thereby completing the present

invention.

The mechanism by which such effects are produced is presumed to be as follows. It should be noted that the following mechanism does not limit the technical scope of the present invention in any way.

Elemental metallic tungsten, due to oxidation by oxygen in the air or during the manufacturing process, is in a state where the oxidation states of metallic tungsten (W) and a very small amount of tungsten oxide ($WO_3$) are mixed, as represented by the following formula. This $WO_3$ comes into contact with water and, through a hydration reaction, becomes tungstic acid, contributing to the release of acidic hydrogen ions.

[Math. 1]

$$W + (3/2)O_2 \rightarrow WO_3$$
$$WO_3 + H_2O \rightarrow H_2WO_4 \rightarrow 2H^+ + WO_4^{2-}$$

Due to the presence of the acidic hydrogen ions, the system tilts to acidic conditions, in other words, the pH value decreases. In fact, 1 g of pelletized resin (resin used in the examples; refer to Table 1, resin only (without tungsten)), 1 g of the sample of Comparative Example 1 (resin + tungsten powder) after being subjected to high temperature and high humidity load for one week (refer to Table 1, resin + tungsten (after one week under high temperature and high humidity load)), and 1 g of the sample of Comparative Example 1 after being subjected to high temperature and high humidity load for one week in a sealed state (refer to Table 1, resin + tungsten (after one week in a sealed state under high temperature and high humidity load)) were respectively added to 5 mL of RO water, stirred, and left to stand at room temperature for 24 hours. The pH was then measured using a pH meter (AS600 manufactured by AS ONE, at 25°C). As a result, as shown in Table 1 below, the sample after one week of high temperature and high humidity load exhibited a lower pH value than the resin-only (without tungsten) sample.

[Table 1]

| **Condition** | **pH** |
|---|---|
| Resin only (without tungsten) | 6.82 |
| Resin + tungsten (after 1 week high temperature and high humidity load) | 5.62 |
| Resin + tungsten (after 1 week high temperature and high humidity load in sealed state) | 6.27 |

Under acidic conditions, ester bond and/or amide bond sites in the resin undergo hydrolysis. Furthermore, as hydrolysis proceeds, the terminal carboxyl groups of the decomposition products act as carboxylic acids, thereby increasing the reaction rate of hydrolysis.

Table 2 below shows the results of molecular weight measurements (in PMMA equivalents) using GPC for the samples of Comparative Example 1 ((1) pre-loaded sample), and the sample left under high temperature and high humidity conditions for a certain period ((2) post-load sample).

[Table 2]

| Condition | **Mn** | **Mw** | **Mz** | **Mw/Mn** |
|---|---|---|---|---|
| (1) Pre-loaded sample | 9248 | 40418 | 102010 | 4.370 |
| (2) Loaded sample | 4429 | 12164 | 22031 | 2.746 |

From these results, it can be understood that in the presence of a tungsten-containing compound, the resin undergoes molecular weight reduction and decomposition due to high temperature and high humidity load. This decomposition of the resin is considered to be due to hydrolysis of the resin.

The progress of such hydrolysis is considered to gradually proceed even under normal temperature and humidity conditions due to long-term storage of the catheter, potentially leading to aging deterioration of the resin.

Here, it is considered that the presence of the hydrolysis inhibitor suppresses the progression of the above-described hydrolysis, and as a result, the resin deterioration is suppressed.

Hereinafter, the catheter according to the present invention will be described with reference to the drawings. In the description of the drawings, the same reference numerals will be given to identical elements, and redundant explanations will be omitted. Dimensional ratios in each drawing may be exaggerated for convenience of description, and might be different from actual ratios.

Furthermore, an aspect in which two or three or more of preferable individual aspects of the present invention

described below are combined is also regarded as a preferable aspect of the present invention and disclosed in the present specification (that is, it constitutes a legitimate basis for amendment).

Fig. 1 is a diagram showing an overall configuration of a catheter 1 of the present invention.

In Fig. 1, the side of the catheter 1 that is inserted into the living body (the left side in Fig. 1) is referred to as the distal end side, the side of the catheter 1 on which a hub 30 is disposed is referred to as the proximal end side, and the direction in which a catheter main body portion 10 of the catheter 1 extends is referred to as the axial direction. Furthermore, in a transverse cross section (cross section orthogonal to the axis) of the catheter main body portion 10 with the axial direction of the catheter main body portion 10 as a reference axis, a direction away from or approaching the catheter main body portion 10 is referred to as a "radial direction".

The catheter 1 includes the catheter main body portion 10 extending in the axial direction, a catheter tip 20 disposed on the distal end side of the catheter main body portion 10, a hub 30 disposed on the proximal end side of the catheter main body portion 10, and a kink-resistant protector (strain relief) 40 disposed between the catheter main body portion 10 and the hub 30. The catheter main body portion 10 and the catheter tip 20 are joined to each other.

The catheter main body portion 10 is formed of a hollow tubular member provided with flexibility. The catheter main body portion 10 has a lumen 10H over the entire length of the catheter main body portion 10. The lumen 10H is open at a distal end of the catheter tip 20.

As shown in Fig. 2, the catheter main body portion 10 includes an inner layer 11 disposed on the inner surface side; an outer layer 12 disposed on the outer periphery of the inner layer 11; a reinforcing material layer 13 disposed inside the outer layer 12; and a hydrophilic lubricating layer 14. In the aspect shown in Fig. 2, the inner layer 11 and the hydrophilic lubricating layer 14 are disposed extending to the distal end of the catheter tip 20. Therefore, the inner layer 11 and the hydrophilic lubricating layer 14 are common to the inner layer and the hydrophilic lubricating layer in the catheter tip 20.

The inner layer 11 is preferably made of a material such that at least the part in contact with devices such as a treatment catheter or a guide wire, which are inserted into the lumen 10H, has low friction. According to this configuration, a device inserted into the catheter main body portion 10 can be moved in the longitudinal direction with lower sliding resistance, thereby improving operability. A specific example of the constituent material for the inner layer 11 includes a fluororesin material such as polytetrafluoroethylene (PTFE).

The outer layer 12 is preferably made of a material that provides kink resistance, suitable pushability, and trackability. Specifically, the constituent material of the outer layer 12 may be polyamide elastomer and/or polyamide (polyamide elastomer, polyamide, or a combination thereof), polyester, polyester elastomer, polyurethane elastomer, polyurethane resin, or a combination of these.

The reinforcing material layer 13 includes a plurality of reinforcing wires that reinforce the catheter main body portion 10. Examples of the reinforcing wires include spiral or braided reinforcing wires. The reinforcing wire is made of metal such as stainless steel. As specific examples, reinforcing wires are formed by crushing stainless steel wires into a flat plate shape to make the thickness of the catheter main body portion 10 thin in the radial direction, and by spiraling or weaving (braided body) a plurality of approximately 8 to 32 stainless steel wires. It is preferable that the number of the reinforcing wires is set to a multiple of 8 to implement reinforcement in a tubular shape in a well-balanced manner.

By making the reinforcing wire into a flat plate, compared to an ellipse, force is received uniformly against external stress, resulting in consistent physical properties.

It should be noted that the number of the layers constituting the catheter main body portion 10 or the constituent material of each layer may be different along the longitudinal direction of the catheter main body portion 10. For example, in order to make the part on the distal end side of the catheter main body portion 10 more flexible, it is possible to reduce the number of layers, to use a more flexible material, to omit a reinforcing material only in the part, or to provide the inner layer up to the distal end.

Insertion of the catheter into the body is performed while confirming the position thereof under X-ray fluoroscopy. In the present invention, since the catheter tip 20 contains the tungsten-containing compound, which is an X-ray opaque material, it is not necessarily required for the catheter main body portion 10 to contain an X-ray opaque material, but an X-ray opaque material (X-ray contrast agent) may be blended into the constituent material of the outer layer 12. As the X-ray opaque material, for example, barium sulfate, bismuth oxide, tungsten, or the like can be used. In addition, the X-ray opaque material is not limited to the case of being present over the entire length of the catheter main body portion 10, and may also be present in a part of the catheter main body portion 10, for example, only in the distal end portion of the catheter main body portion 10 or only in the catheter tip 20.

The constituent material of the hydrophilic lubricating layer 14 is not particularly limited, but examples thereof include: copolymers of epoxy group-containing monomers such as glycidyl acrylate, glycidyl methacrylate, 3,4-epoxycyclohexylmethyl acrylate, 3,4-epoxycyclohexylmethyl methacrylate, β-methylglycidyl methacrylate, and allyl glycidyl ether, with hydrophilic monomers such as N-methylacrylamide, N,N-dimethylacrylamide, and acrylamide; (co)polymers composed of the above hydrophilic monomers; cellulose-based polymer substances such as hydroxypropyl cellulose and carboxymethyl cellulose; polysaccharides, polyvinyl alcohol, methyl vinyl ether-maleic anhydride copolymers, water-soluble polyamides, poly(2-hydroxyethyl (meth)acrylate), polyethylene glycol, polyacrylamide, polyvinylpyrrolidone, and

copolymers of polyvinylpyrrolidone and polyurethane described in U.S. Patent No. 4100309 and Japanese Unexamined Patent Publication No. 59-19582. These hydrophilic lubricating materials may be used singly or in the form of a mixture of two or more types thereof.

The hub 30 is mounted (fixed) to the proximal end of the catheter main body portion 10. In the hub 30, a lumen is formed which communicates with the lumen 10H and in which a Luer taper is formed.

The distal end portion of the catheter main body portion 10 and the catheter tip 20 can be joined, for example, by thermal fusion, thereby being integrated.

From the hub 30, for example, elongated objects (linear bodies) such as guide wires, catheters (for example, balloon catheters, stent delivery catheters), endoscopes, ultrasound probes, temperature sensors, and the like can be inserted or withdrawn, and various liquids such as contrast agents (X-ray contrast agents), medicinal solutions, and physiological saline can be injected. In addition, the hub 30 can also be connected to other instruments, such as a Y-shaped branch connector. Furthermore, examples of a constituent material for the hub 30 include a thermoplastic resin such as polycarbonate, polyamide, polysulfone, or polyarylate.

Hereinafter, the catheter tip, the constituent material thereof, and the like will be described. In the present specification, a range of "X to Y" includes X and Y, and indicates "X or more and Y or less". In addition, unless otherwise specified, operations and measurements of physical properties and the like are performed at room temperature (20 to 25°C) and at relative humidity of 40 to 50% RH.

(Catheter Tip)

The catheter tip 20 is formed of a material softer than the catheter main body portion 10. The catheter tip 20 is also referred to as a distal end tip, and has a function of suppressing damage to a lumen in a living body such as a blood vessel and a function of improving insertability into a stenosed site formed in the blood vessel. In Fig. 2, the catheter tip 20 has a tapered shape with a decreasing outer diameter toward the distal end side, but the outer diameter may be identical up to the distal end side.

The catheter tip includes a resin having an ester bond and/or an amide bond (a resin having an ester bond, an amide bond, or a combination thereof); a tungsten-containing compound; and a hydrolysis inhibitor. Since the catheter tip contains a tungsten-containing compound, the catheter tip has X-ray contrast ability. The resin having an ester bond and/or an amide bond, the tungsten-containing compound, and the hydrolysis inhibitor are preferably present in the same layer (tungsten-containing layer (in the catheter tip, a layer containing the tungsten-containing compound is also referred to as a tungsten-containing layer)). In addition, the tungsten-containing layer may be a single layer or a plurality of layers of two or more layers. In the case of a plurality of layers, the composition (resin type, mixing ratio, or the like) may be different or the same. Furthermore, the tungsten-containing compound is preferably dispersed as particles within the resin.

The catheter tip may consist of only the tungsten-containing layer, or other functional layers (for example, the hydrophilic lubricating layer described above) may be laminated on the inner layer side (lumen side) and/or the outer layer side (surface side) of the tungsten-containing layer.

Examples of resins having an ester bond and/or an amide bond (hereinafter also simply referred to as resins having a bond) include ethylene-vinyl acetate copolymer, polyamide, polyamide elastomer, polyurethane elastomer, and polyamide-imide. The above resin may be used singly, or two or more types may be laminated or blended.

Among these, the resin having a bond is preferably one that includes a polyamide-based resin due to its high flexibility and high affinity or low hardness difference with adjacent members; more preferably includes polyamide and/or polyamide elastomer (polyamide, polyamide elastomer, or a combination thereof); and even more preferably includes a polyamide elastomer. Among the resins having a bond, the polyamide-based resin is preferably 50% by mass or more (upper limit: 100% by mass), more preferably 80% by mass or more (upper limit: 100% by mass), and even more preferably 100% by mass (composed of a polyamide-based resin). Further, among the resins having a bond, the polyamide elastomer is preferably 50% by mass or more (upper limit: 100% by mass), more preferably 80% by mass or more (upper limit: 100% by mass), and even more preferably 100% by mass (composed of a polyamide elastomer).

In addition, among the resins constituting the catheter tip, the resin having a bond is preferably 80% by mass or more (upper limit: 100% by mass), more preferably 90% by mass or more (upper limit: 100% by mass), and even more preferably 100% by mass (composed of resin having a bond). Further, among the resins in the tungsten-containing layer, the resin having a bond is preferably 80% by mass or more (upper limit: 100% by mass), more preferably 90% by mass or more (upper limit: 100% by mass), and even more preferably 100% by mass (composed of resin having a bond).

The polyamide elastomer is a thermoplastic resin composed of a copolymer having a hard segment derived from a polymer that is crystalline and has a high melting point and a soft segment derived from a polymer that is amorphous and has a low glass transition temperature, and refers to one in which the main chain of the polymer forming the hard segment has an amide bond (-CONH-). A structural unit having an amide bond (-CONH-) in the main chain of the polymer forming the hard segment is also referred to as an amide unit of the polyamide elastomer.

The "amide unit of the polyamide elastomer" in the present specification refers to a repeating unit derived from an

amide bond in the polyamide elastomer polymer chain, and the amide unit in the polyamide elastomer is preferably a repeating unit represented by the following Formula (1).

[Chem. 1]

Formula (1)

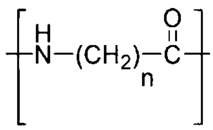

In Formula (1) above, n is preferably an integer of 2 to 20, and more preferably an integer of 5 to 11. Note that n is 11 in the examples described below.

Examples of the polymer forming the soft segment include polyester and polyether. Moreover, examples thereof include polyethers such as polyethylene glycol, polypropylene glycol, polytetramethylene ether glycol (PTMG), and polyester polyol, and ABA-type triblock polyether diols. The polymer forming the soft segment may be used singly or in combination of two or more types thereof. Furthermore, a polyether diamine or the like obtained by reacting ammonia or the like with the terminal of polyether can be used, and for example, an ABA-type triblock polyether diamine can be used. The polyether, which is a polymer capable of forming a soft segment, can form a polyether block amide copolymer in which the polyether block is bonded to the polyamide block, which is a hard segment, via an ester bond.

The content ratio of the soft segment in the polyamide elastomer is preferably 1 to 50% by mass, and more preferably 10 to 30% by mass.

As the polyamide elastomer, a chain extender such as a dicarboxylic acid may be used in addition to the hard segment and the soft segment.

These polyamide elastomers may be used singly or in combination of two or more types thereof.

The weight average molecular weight of the polyamide elastomer is preferably 10,000 to 500,000, more preferably 15,000 to 400,000, and still more preferably 20,000 to 300,000.

The molecular weight of the polymer according to the present invention can be measured by a known method such as mass spectrometry, light scattering, liquid chromatography, gas chromatography, or gel filtration chromatography (GPC), and in the present specification, the molecular weight is measured by GPC.

As measurement conditions for the GPC method, for example, the following conditions can be mentioned.

(1) Pretreatment: filtration through a 0.45 μm PTFE cartridge filter
(2) Equipment: HLC-8420GPC (manufactured by Tosoh)
(3) Separation column: TSKgel Super AWM-H (6.0 mm I.D. x 15 cm)
(4) Measurement temperature: 40°C
(5) Carrier: hexafluoroisopropanol (+10 mM $CF_3COONa$)
(6) Flow rate: 0.3 mL/min
(7) Injection volume: 20 μL
(8) Detector: differential refractometer (RI detector), polarity = (+)
(9) Concentration: 1 mg/mL
(10) Molecular weight standard: standard polymethyl methacrylate

The Shore D hardness of the polyamide elastomer used for the catheter tip is preferably 20 to 80 and more preferably 30 to 50 from the viewpoint of flexibility. As a method of measuring hardness of the polyamide elastomer, the Shore D hardness according to ISO 868:2003 is used. When a plurality of kinds of polyamide elastomers are used, the Shore D hardness of the whole polyamide elastomer in consideration of the content mass ratio is used.

Polyamides are not particularly limited as long as the polyamides are polymers having an amide bond (-CO-NH-) in the main chain, and are typically produced by polymerization of lactams having a cyclic structure or amino acids, or by polycondensation of dicarboxylic acids and diamines. As the polyamide, a homopolyamide is preferably used. Examples of monomers capable of polymerizing alone include ε-caprolactam, ω-laurolactam, 6-aminocaproic acid, enantholactam, 7-aminoheptanoic acid, 11-aminoundecanoic acid, 12-aminododecanoic acid, 9-aminononanoic acid, and piperidone.

In addition, in the polycondensation of a dicarboxylic acid and a diamine, examples of the dicarboxylic acid include adipic acid, sebacic acid, dodecane dicarboxylic acid, glutaric acid, terephthalic acid, 2-methylterephthalic acid, isophthalic acid, and naphthalenedicarboxylic acid. Examples of diamines include tetramethylenediamine, hexamethylenediamine, nonamethylenediamine, decamethylenediamine, undecamethylenediamine, dodecamethylenediamine,

para-phenylenediamine, and meta-phenylenediamine.

Examples of the polyamide include NYLON 4, 6, 7, 8, 11, 12, 6.6, 6.9, 6.10, 6.11, 6.12, 6T, 6/6.6, 6/12, 6/6T, and 6T/6I. The polyamide can be used singly or in combination of two or more types thereof.

In addition, the terminal of the polyamide may be capped with a carboxyl group, amine, or the like. Examples of carboxylic acids include aliphatic monocarboxylic acids such as caproic acid, caprylic acid, capric acid, lauric acid, myristic acid, palmitic acid, stearic acid, and behenic acid. Furthermore, examples of the amine include aliphatic primary amines such as hexylamine, octylamine, decylamine, laurylamine, myristylamine, palmitylamine, stearylamine, and behenylamine.

The weight average molecular weight of the polyamide is preferably 10,000 to 500,000, more preferably 15,000 to 400,000, and still more preferably 20,000 to 300,000.

The content of the resin having a bond relative to the total amount (100% by mass) of the resin having a bond, the tungsten-containing compound, and the hydrolysis inhibitor is appropriately set in consideration of factors such as X-ray contrast ability, flexibility, and moldability, and may be, for example, 10 to 90% by mass, or 20 to 70% by mass.

(Tungsten-Containing Compound)

The catheter tip contains tungsten-containing compound as an X-ray opaque material. Examples of tungsten-containing compounds include elemental metallic tungsten and tungsten carbide. The tungsten-containing compound may be used singly or in combination of two or more types thereof.

Furthermore, the tungsten-containing compound is preferably in the form of particles. Here, for example, tungsten carbide particles refer to those in which tungsten carbide is the main component (the most abundant component) of the particles (the main component is preferably 80% by mass or more, 90% by mass or more, 95% by mass or more, and 98% by mass or more), and typically include uncarbided tungsten within the tungsten carbide.

As the tungsten-containing compound, tungsten powder (particles) is preferable. Typically, tungsten powder contains tungsten (W) as the main component (the main component is preferably 80% by mass or more, 90% by mass or more, 95% by mass or more, and 98% by mass or more), and in addition, may include oxygen (forming trace amounts of tungsten oxides such as $WO_{X'}$ ($X' \approx 2$) and $WO_X$ ($2 < X < 3$) bonded with tungsten), as well as iron (Fe), molybdenum (Mo), and the like.

The average particle diameter of the tungsten powder is preferably 0.5 to 30 μm, more preferably 1 to 10 μm, and even more preferably 2 to 4 μm. The average particle diameter is a volume average particle diameter measured by dynamic light scattering, but is not limited thereto.

In addition, various types of tungsten powder are commercially available, and these commercial products can be preferably used in the present invention.

The content of the tungsten-containing compound is preferably 10 to 90% by mass, more preferably 20 to 80% by mass, and even more preferably 50 to 80% by mass, and may also be more than 50% by mass and 80% by mass or less, and may be 60 to 80% by mass, relative to the total amount of the resin having a bond and the tungsten-containing compound. When the content of the tungsten-containing compound is within the above range, excellent contrast ability and flexibility can be achieved.

(Hydrolysis Inhibitor)

As the above hydrolysis inhibitor, conventionally known compounds can be used. Examples thereof include compounds that react and bond with the carboxyl group at the terminal of decomposition products. Specifically, compounds containing functional groups such as a carbodiimide group, an epoxy group (for example, glycidyl ester compounds and glycidyl ether compounds), and an oxazoline group (for example, bisoxazoline compounds), and the like may be mentioned. Among them, from the viewpoint of hydrolysis inhibition effect, it is preferable that the hydrolysis inhibitor is a carbodiimide group-containing compound.

The carbodiimide group-containing compound is a compound having a carbodiimide group (-N=C=N-) in the molecule. The carbodiimide group-containing compound is preferably polyfunctional (having two or more carbodiimide groups in the molecule).

In addition, the carbodiimide group-containing compound is preferably cyclic carbodiimide or polycarbodiimide. Among them, the carbodiimide group-containing compound is preferably polycarbodiimide because the effect of the present invention is further exhibited.

Examples of the polycarbodiimide include an aromatic polycarbodiimide compound, an aliphatic polycarbodiimide compound, and an alicyclic polycarbodiimide compound. Here, the polycarbodiimide is usually linear. The aromatic polycarbodiimide compound is a polycarbodiimide compound which has an aromatic ring in the molecule and may or may not have an aliphatic ring. The aliphatic polycarbodiimide compound refers to a polycarbodiimide compound that does not have an aromatic ring or an aliphatic ring in the molecule. The alicyclic polycarbodiimide compound refers to a

polycarbodiimide compound that has an aliphatic ring but does not have an aromatic ring in the molecule. Among them, from the viewpoint of storage stability and the like, an aromatic polycarbodiimide compound and/or an alicyclic polycarbodiimide compound are preferable, an alicyclic polycarbodiimide compound is more preferable, and a cyclic polycarbodiimide compound may be used.

Specific examples of polycarbodiimides include those obtained by decarboxylative condensation of diisocyanates. The diisocyanate may be, for example, any of a chain or alicyclic aliphatic diisocyanate compound, an aromatic diisocyanate compound, or a heterocyclic diisocyanate compound, and one of these may be used singly or in combination of two or more types thereof. Specific examples include linear aliphatic diisocyanates such as tetramethylene diisocyanate, hexamethylene diisocyanate, dodecamethylene diisocyanate, and 2,2,4-trimethylhexamethylene diisocyanate; alicyclic diisocyanates such as 1,4-bis(isocyanatomethyl)cyclohexane, 2,2-bis(4-isocyanatocyclohexyl)propane, isophorone diisocyanate, and dicyclohexylmethane-4,4'-diisocyanate; aliphatic diisocyanates containing an aromatic ring such as 1,3-bis(2-isocyanato-2-propyl)benzene; and aromatic isocyanates such as toluene-2,4-diisocyanate, 2,2'-diphenylmethane diisocyanate, 2,4'-diphenylmethane diisocyanate, 4,4'-diphenylmethane diisocyanate, and 2,4,6-triisopropylbenzene-1,3-diyl diisocyanate. These may be used singly or in combination of two or more kinds thereof.

The polycarbodiimide is preferably one in which the terminal isocyanate group is sealed with a sealant from the viewpoint of storage stability. Examples of the sealant include a compound having active hydrogen that reacts with an isocyanate group and a compound having an isocyanate group. Examples thereof include monoalcohols, monocarboxylic acids, monoamines, and monoisocyanates each having one substituent selected from a carboxy group, an amino group, and an isocyanate group.

Examples of cyclic carbodiimides include cyclic carbodiimide compounds represented by Formula (i) disclosed in International Publication No. WO 2010/071211 (specifically, the cyclic carbodiimide compounds described on pages 13 to 15, and the cyclic carbodiimide compounds represented by Formula (i) disclosed in U.S. Patent Application Publication No. 2011/251384 (pages 4 to 6), of which the description is incorporated herein by reference (Incorporation by Reference)), and cyclic carbodiimide compounds represented by Formula (i) described in Japanese Unexamined Patent Application Publication No. 2016-65001 (specifically, the cyclic carbodiimide compounds described on pages 10 to 11).

The method for adding and mixing the hydrolysis inhibitor with the resin is not particularly limited, and the hydrolysis inhibitor may be mixed by dry blending, or the resin may be brought into a solution or molten state, and the hydrolysis inhibitor (for example, a carbodiimide group-containing compound) may be kneaded. In addition, a solvent may be used at the time of kneading. As the solvent, for example, a hydrocarbon-based solvent, a ketone-based solvent, an ester-based solvent, an ether-based solvent, a halogen-based solvent, an amide-based solvent, or the like can be used.

The addition amount of the hydrolysis inhibitor is preferably 2 parts by mass or less, more preferably 0.01 to 2 parts by mass, and still more preferably 0.05 to 1 part by mass relative to 100 parts by mass of the resin having a bond. When the addition amount of the hydrolysis inhibitor is within the above range, resin deterioration is effectively suppressed, and the characteristics of the resin are hardly affected.

The catheter tip 20 may be provided with a hydrophilic lubricating layer that imparts surface lubricity when wet (hydrophilic lubricating layer 14 in Fig. 2). The hydrophilic lubricating layer that imparts surface lubricity when wet is disposed radially outward of the tungsten-containing layer.

The constituent material of the hydrophilic lubricating layer is not particularly limited, but examples include: copolymers of epoxy group-containing monomers such as glycidyl acrylate, glycidyl methacrylate, 3,4-epoxycyclohexylmethyl acrylate, 3,4-epoxycyclohexylmethyl methacrylate, β-methylglycidyl methacrylate, and allyl glycidyl ether with hydrophilic monomers such as **N-**methylacrylamide, N,N-dimethylacrylamide, and acrylamide; (co)polymers composed of the above hydrophilic monomers; cellulose-based polymer substances such as hydroxypropyl cellulose and carboxymethyl cellulose; polysaccharides, polyvinyl alcohol, methyl vinyl ether-maleic anhydride copolymer, water-soluble polyamide, poly(2-hydroxyethyl (meth)acrylate), polyethylene glycol, polyacrylamide, polyvinylpyrrolidone, and copolymers of polyvinylpyrrolidone and polyurethane as described in U.S. Patent No. 4100309 and Japanese Unexamined Patent Publication No. 59-19582. These hydrophilic lubricating materials may be used singly or in the form of a mixture of two or more types thereof.

Further, the catheter tip 20 may be provided with an inner layer such that at least the part which is in contact with devices has low friction when treatment catheters or guide wires are inserted into the lumen 10H (the inner layer 11 in Fig. 2). The inner layer is preferably made of a material such that at least the part which is in contact with devices has low friction when treatment catheters or guide wires are inserted into the lumen 10H. Specifically, examples of the constituent material of the inner layer include a fluororesin material such as polytetrafluoroethylene (PTFE).

The catheter tip may include a pigment or dye that imparts a color such as white, black, blue, red, or yellow, or a mixture thereof. Examples of such pigments or dyes include carbon black, activated carbon, graphite, carbon nanotubes, fullerenes; titanium oxide; condensed polycyclic organic pigments such as cyanine-based pigments, nickel dithiolene-based pigments, squarylium-based pigments, naphthoquinone-based pigments, diimonium-based pigments, azo-based organic pigments, phthalocyanine-based pigments, naphthalocyanine-based pigments, and azulenocyanine-based pigments.

In addition, in the catheter tip, a reinforcing body made of SUS or the like may be disposed in the above-described material. The reinforcing body may have coil-shaped and braid-shaped aspects.

Examples

The effects of the present invention will be described with reference to the following Examples and Comparative Examples. Note that the technical scope of the present invention is not limited only to the following Examples. Note that, in the following Examples, unless otherwise specified, an operation was performed at room temperature (25°C). In addition, unless otherwise specified, "%" and "parts" mean "% by mass" and "parts by mass", respectively.

[Preparation of Dumbbell-Shaped Test Pieces]

[Example 1]

A mixture was prepared by blending 30 parts by mass of a polyamide elastomer (Shore D hardness: 40, weight average molecular weight (Mw): approximately 40,000), 70 parts by mass of tungsten particles (average particle diameter: 3.0 μm, W content: 99.9% or more), and 0.3 parts by mass of an alicyclic polycarbodiimide compound which is a carbodiimide group-containing compound as a hydrolysis inhibitor. Press molding was performed using a 20-ton manual hydraulic heating press at a set temperature of 200°C. The thickness of the molded product was set to 1.9 to 2.1 mm. The molded press sheet was punched into a dumbbell-shaped test piece according to JIS K 7161-2:2014 5A.

[Example 2]

Except for using a cyclic carbodiimide compound which is a carbodiimide group-containing compound instead of the alicyclic polycarbodiimide compound as the hydrolysis inhibitor, a dumbbell-shaped test piece was prepared in the same manner as in Example 1.

[Comparative Example 1]

Except for not using a carbodiimide group-containing compound as the hydrolysis inhibitor, a dumbbell-shaped test piece was prepared in the same manner as in Example 1.

[Load Test]

The dumbbell-shaped test pieces prepared in the examples and comparative examples were left under high temperature and high humidity conditions. Specifically, a load test was conducted on the dumbbell-shaped test piece under the conditions described in Table 3 below, with one cycle defined as 24 hours, for a total of 7 cycles (one week).

[Table 3]

Table 3: Cycle load conditions (1 cycle = 24 hours, excluding transition time)

| Step No. | 1 | 2 | 3 | 4 | 5 | 6 |
|---|---|---|---|---|---|---|
| Temperature [°C] | 65 | 25 | 65 | 25 | -10 | 25 |
| Humidity [% RH] | 95 | 65 | 65 | 65 | | 65 |
| Duration [hr.] | 5.5 | 5.0 | 5.5 | 2.0 | 4.0 | 2.0 |

[Tensile Test]

A tensile test was conducted on the dumbbell-shaped test pieces of Examples 1 and 2 and Comparative Example 1 before and after the load test in accordance with JIS K 7161-1:2014.

<Examination Conditions>

Distance between grips: 50 mm
Distance between marked lines: 20 mm

Test speed: 100 mm/min

As an indicator of durability, the residual ratio shown in the following formula was calculated. The higher the residual ratio, the greater the durability. Here, the fracture stroke length refers to the value obtained by subtracting the gauge length before the test from the distance between the marked lines after fracture.

[Math. 2]

$$\text{Residual ratio (\%)} = B/A \times 100$$

A: Fracture stroke length before load test (mm)
B: Fracture stroke length after load test (mm)

[Table 4]

Table 4: Tensile test results of Examples 1 and 2 and Comparative Example 1 before and after load test

| | Fracture Stroke Length [mm] | | Residual ratio [%] = B/A |
|---|---|---|---|
| | No load (A) | After 7 cycles (B) | |
| Example 1 | 285.1 | 148.1 | 52.0 |
| Example 2 | 330.9 | 43.4 | 13.1 |
| Comparative Example 1 | 267.4 | 18.9 | 7.1 |

As a result of the tensile test, the dumbbell-shaped test piece of Comparative Example 1 showed a residual ratio of 7% before and after the load test, whereas the dumbbell-shaped test piece of Examples 1 and 2 showed a higher residual ratio of 10% or more before and after the load test, indicating a higher residual ratio than that of Comparative Example 1. From this result, it was confirmed that the dumbbell-shaped test piece of Example 1 had higher durability than the dumbbell-shaped test piece of Comparative Example 1.

The present application is based on Japanese Patent Application No. 2023-081659 filed on May 17, 2023, the entire content of which is incorporated herein by reference.

Reference Signs List

1 Catheter
10 Catheter main body portion
30 Hub
20 Catheter tip
40 Kink-resistant protector (strain relief)

## Claims

1. A catheter tip comprising: a resin having an ester bond and/or an amide bond; a tungsten-containing compound; and a hydrolysis inhibitor.

2. The catheter tip according to claim 1, wherein the hydrolysis inhibitor is a carbodiimide group-containing compound.

3. The catheter tip according to claim 1 or 2, wherein the content of the tungsten-containing compound is 10 to 90% by mass relative to a total amount of the resin and the tungsten-containing compound.

4. The catheter tip according to claim 1 or 2, wherein the resin having an ester bond and/or an amide bond includes a polyamide-based resin.

5. The catheter tip according to claim 1 or 2, wherein the tungsten-containing compound is dispersed as particles within

the resin.

**6.** A catheter comprising the catheter tip according to claim 1 or 2.

FIG. 1

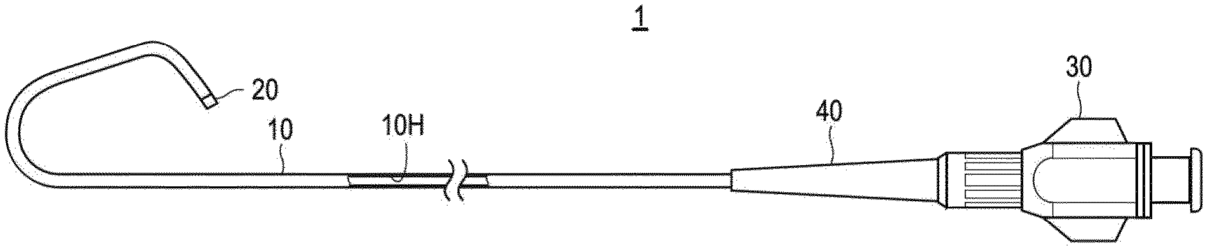


FIG. 2

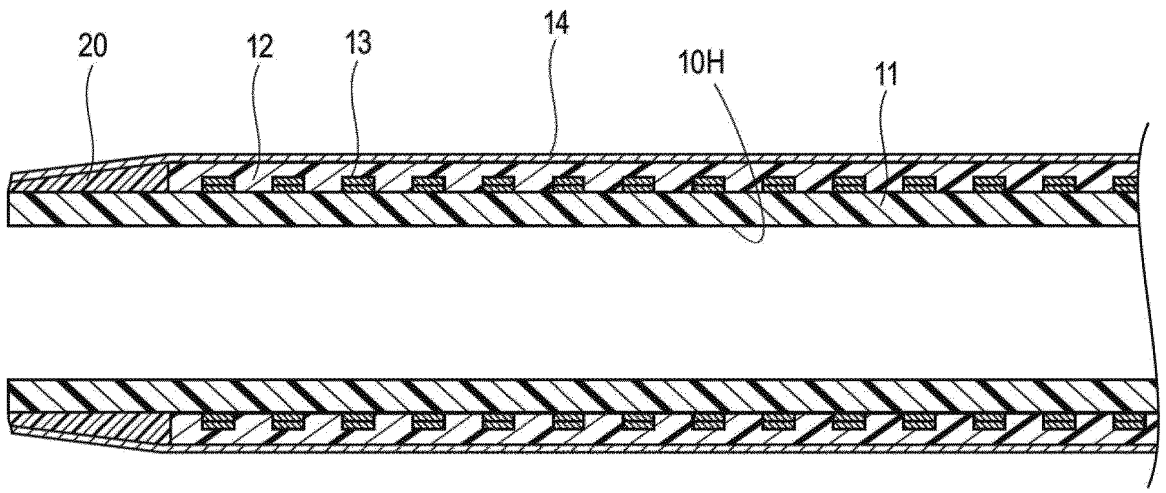

# INTERNATIONAL SEARCH REPORT

International application No.
**PCT/JP2024/016709**

## A. CLASSIFICATION OF SUBJECT MATTER

***A61L 29/02***(2006.01)i; ***A61L 29/06***(2006.01)i; ***A61L 29/14***(2006.01)i; ***C08K 3/11***(2018.01)i; ***C08K 5/29***(2006.01)i; ***C08L 77/00***(2006.01)i
FI: A61L29/02; A61L29/06; A61L29/14 200; C08K3/11; C08K5/29; C08L77/00

According to International Patent Classification (IPC) or to both national classification and IPC

## B. FIELDS SEARCHED

Minimum documentation searched (classification system followed by classification symbols)

A61L29/02; A61L29/06; A61L29/14; C08K3/11; C08K5/29; C08L77/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

## C. DOCUMENTS CONSIDERED TO BE RELEVANT

| Category* | Citation of document, with indication, where appropriate, of the relevant passages | Relevant to claim No. |
|---|---|---|
| X | US 2017/0313853 A1 (MEDTRONIC, INC.) 02 November 2017 (2017-11-02) claims 1-9, paragraphs [0001], [0120], examples 1-19, 4 | 1-6 |
| X | US 2010/0130962 A1 (EBERT, Michael J.) 27 May 2010 (2010-05-27) claims 1, 8, paragraphs [0011], [0021], [0038] | 1-6 |
| X | US 2010/0130963 A1 (EBERT, Michael J.) 27 May 2010 (2010-05-27) claims 1, 10, paragraphs [0013], [0023], [0041] | 1-6 |

☐ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 July 2024** | **16 July 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/016709**

| Patent document cited in search report | Publication date (day/month/year) | Patent family member(s) | Publication date (day/month/year) |
|---|---|---|---|
| US 2017/0313853 A1 | 02 November 2017 | WO 2017/189761 A1<br>EP 3448449 A1<br>CN 109069700 A | |
| US 2010/0130962 A1 | 27 May 2010 | WO 2010/059408 A2<br>EP 2376137 A2 | |
| US 2010/0130963 A1 | 27 May 2010 | WO 2010/059409 A2<br>EP 2373357 A2 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5584821 A
- US 4100309 A
- JP 59019582 A
- WO 2010071211 A
- US 2011251384
- JP 2016065001 A
- JP 2023081659 A